# EUROPEAN PATENT SPECIFICATION

(11) **EP 1 748 353 B1**
(45) Date of publication and mention of the grant of the patent: **01.07.2009**
(21) Application number: 05016470.6
(22) Date of filing: 29.07.2005
(51) Int. Cl.: G06F 3/033, A61L 2/238

(54) **Touch screen with bacteria inhibition layer**
Berührungsfühlsamer Schirm mit antibakterieller Schicht
L'écran tactile avec une couche anti-bactérienne

(43) Date of publication of application: 31.01.2007
(73) Proprietor: TPK Touch Solutions Inc., Taipei City (TW)
(72) Inventor: Hu, Chun-Min, Keelung (TW)
(74) Representative: Berngruber, Otto

(56) References cited:
- EP-A- 0 942 351
- WO-A-00/43831
- WO-A-01/46900
- DE-A1- 10 120 802
- US-A1- 2005 058 835

## Description

The present invention relates to touch screens and more particularly to a method of manufacturing such a touch screen having a bacteria inhibition layer for prohibiting bacteria from growing thereon.

### BACKGROUND OF THE INVENTION

Electronics and computer technologies have made a significant progress in recent years due to booming of the Internet. Many compact, portable products are developed by electronic and computer product designers and manufacturers for fulfilling the urgent needs of vast consumers. Moreover, a number of significant, revolutionary modifications have been made with respect to input component or output component of any one of the products due to the consideration of user friendliness. The most important one of the modifications is the development of touch screen. In one aspect, a touch screen as a component of an electronic and computer product is adapted to show output characters or graphics thereon. In another aspect, the touch screen is adapted to send characters or instructions inputted by a user to the electronic and computer product. In other words, a touch screen serves as both input and output devices of an electronic and computer product. After viewing description or icons shown on a touch screen of an electronic and computer product, a user can point to things on it by touching a button or icon on the screen itself so as to operate the electronic and computer product. Thus, a user can not only use the electronic and computer product conveniently but also operate the same in a user friendly manner. For a software developer, the electronic and computer product provides a flexible, interactive operating platform in developing software with user friendly feature. For a consumer, no additional input device is required to install in the electronic and computer product. Thus, expense for buying an input device is saved and there is no need of preserving space on the electronic and computer product.

Recently, many types of electronic and computer products with touch screen are widely installed in public places such as schools, department stores, hospitals, airports, and railroad stations. Any person thus can touch the screen of a touch screen for information inquiry and guide. For users, touch screen is an input device with simple operation and user friendly features. Moreover, most problems encountered by people in public places can be solved successfully if software installed in a touch screen is well designed. However, bacteria may grow on the touch screen of an electronic and computer product since it is installed in a public place for user operation by touching the screen. This is a great threat to hygiene and public health. Thus, how to maintain a clean touch screen of one of many types of electronic and computer products installed in public places is a critical, important issue for authority of each public place.

In view of the fact that touch screens have become medium of disseminating bacteria thus for solving this problem many designers and manufacturers of electronic and computer products have developed a touch screen with bacteria inhibition feature in which a bacteria inhibition layer is coated on the touch screen. Typically, the bacteria inhibition layer is formed of organic material as implemented by designers and manufacturers of electronic and computer products. Organic material coated on a touch screen can inhibit growth of bacteria. However, organic material has a low melting point or boiling point and is easy to evaporate or decompose. Thus, its bacteria inhibition capability only lasts for a short period of time. Moreover, generally speaking, organic material is toxic. Hence, it is not appropriate to coat organic material on a product designed to be touched by users. Recently, still some manufacturers of the art employ popular titanium dioxide as optical catalyst for inhibiting growth of bacteria or even destroying the same. Titanium dioxide is subjected to UV (ultraviolet)-light for being catalyzed and thus for destroying bacteria. However, UV-light is very weak in a room environment. Thus, the desired bacteria inhibition effect is substantially compromised.

EP-0 942 351 discloses a method of making a touch screen with a glass based screen antimicrobial by application of a silver containing coating.

Thus, it is desirable to choose a suitable bacteria inhibition material and provide a novel process of manufacturing touch screens with a bacteria inhibition layer in order to contribute significantly to the advancement of the art.

### SUMMARY OF THE INVENTION

After considerable research and experimentation, a manufacturing method for producing a touch screen with a bacteria inhibition layer according to the present invention as defined in claim 1 has been devised so as to overcome the above drawback of the prior art.

It is an object of the present invention to provide a method for manufacturing a touch screen comprising uniformly dispersing particles of nano metal material in a solution to be applied to a surface treatment so that the solution can have a concentration of 20ppm to 500ppm; evenly spray coating the solution on a screen of the touch screen; and subjecting the solution coated on the screen of the touch screen to a heat treatment until solvent in the solution is completely evaporated so that the particles of the nano metal material are densely adhered to the screen of the touch screen to form a bacteria inhibition layer thereon.

It is part of the present invention to subject a resistive touch screen, to be touched by users to a prior surface treatment (e.g., hardening treatment, endurability treatment, anti-glare treatment, or anti-reflection treatment) and the process of the present invention comprises uniformly dispersing particles of nano metal material in a solution to be applied to the surface treatment so that the solution can have a concentration of 20ppm to 500ppm; evenly spray coating the solution on the screen of the touch screen; and subjecting the solution coated on the screen of the touch screen to a heat treatment until the particles of the nano metal material contained are densely adhered to the screen of the touch screen to form a bacteria inhibition layer thereon.

It is another object of the present invention to, in preparing the solution, employ a milling method or an ultrasonic method to uniformly disperse the particles of the nano metal material in the solution to be applied to the surface treatment.

It is yet another object of the present invention to employ a spin coating, a dipping coating, a spray coating, or a rolling coating to evenly spray coating the solution on the screen of the touch screen.

The substrate for the touch screen is PET film. It is a further object of the present invention to provide the solution for the surface treatment including UV-light resin or thermosetting resin and appropriate solvent such that after evenly spray coating the solution on a surface of the PET film, it is subjected to UV lamp radiation or heat treatment depending on types of resin being used wherein the heat treatment is done in a relative low temperature, for example, between about 50°C and 100°C.

The above and other objects, features and advantages of the present invention will become apparent from the following detailed description taken with the accompanying drawings.

### BRIEF DESCRIPTION OF THE DRAWINGS

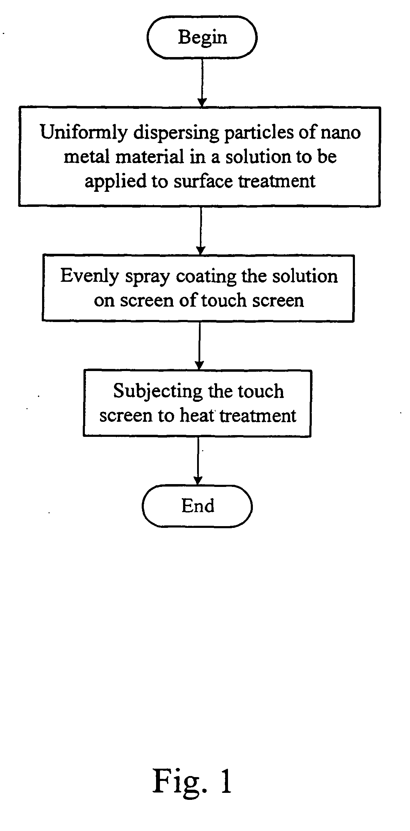

FIG. 1 is a flowchart depicting a process for manufacturing touch screen with bacteria inhibition layer according to the invention.

### DETAILED DESCRIPTION OF THE PREFERRED EMBODIMENTS

Referring to FIG. 1, a process for manufacturing touch screen with a bacteria inhibition layer in accordance with the invention is illustrated. The process comprises uniformly dispersing particles of nano metal material having a diameter in the range of about 1 nm to 100nm in a solution to be applied to surface treatment so that the solution can have a concentration of 20ppm to 500ppm; evenly spray coating the solution on a screen of a touch screen; and subjecting the solution coated on the touch screen to a heat treatment until solvent in the solution is completely evaporated. As such, particles of nano metal material are densely adhered to the screen of touch screen to form a bacteria inhibition layer thereon. As a result, a touch screen with a bacteria inhibition layer is produced. Nano metal material as defined by the invention means that one has biochemical activation, is able to penetrate cell membrane of a bacteria for damaging enzyme and killing the same naturally, and has particles having a diameter in the range of about 1nm to 100nm. Nano metal material may be selected from nano gold (Au), nano silver (Ag), nano copper (Cu), nano zinc (Zn), nano platinum (Pt), or a combination or compound thereof such as nano silver oxide, nano copper oxide, nano zinc oxide, nano silver nitrate, nano copper nitrate, or nano zinc nitrate.

In the invention, the touch screen is a resistive one. The screen of the touch screen to be touched by users is subjected to an appropriate surface treatment depending on applications. For example, surface treatment can be a hardening treatment, endurability treatment, anti-glare treatment, or anti-reflection treatment. In the invention the process comprises uniformly dispersing particles of nano metal material in a solution to be applied to surface treatment so that the solution can have a concentration of 20ppm to 500ppm. Next, the process comprises evenly spray coating the solution on a screen of a touch screen by employing a conventional coating method such as spin coating, dipping coating, spray coating, or rolling coating. Next, the process comprises subjecting the solution coated on the touch screen to a heat treatment until solvent in the solution is completely evaporated. As such, particles of nano metal material contained in the solution are densely adhered to the screen of touch screen to form a bacteria inhibition layer thereon. As a result, a touch screen with a bacteria inhibition layer is produced. In the step of preparing the solution, a milling method or ultrasonic method can be employed to uniformly disperse particles of nano metal material in the solution.

Conventionally, the substrates for manufacturing a touch screen can be classified as an organic compound on an inorganic compound based on properties of material. According to the invention the substrate is a PET (polyethylene terephthalate) film. In a case of the substrate for the touch screen being PET film, the solution for the surface treatment comprises UV-light (or thermosetting) resin and appropriate solvent. After evenly spray coating the solution on a surface of PET film, it is subjected to UV lamp radiation or heat treatment depending on types of resin being used in which the heat treatment is done in a relative low temperature, for example, between about 50°C and 100°C.

A couple of embodiments will be described in detail below for fully describing design spirit and operating principle of the invention as contemplated by the present inventor. Also, an experiment is conducted with respect to the produced touch screen so as to fully demonstrate bacteria inhibition or destroying effect of the invention.

In the invention the touch screen is a resistive one having its substrate formed of PET film. In a hardening treatment it is possible of uniformly dispersing particles of nano silver weighted 0.1g and having a diameter of about 10nm in methyl ethyl ketone weighted 100g for preparing a required dispersing solution; adding an UV hardener mainly consisting of polybutene acrylate monomers weighted 900g in the dispersing solution; uniformly mixing them; evenly spray coating the solution on screen of the resistive touch screen; subjecting the screen of the resistive touch screen to an IR heating for about 5 minutes until solvent in the solution is completely evaporated; and subjecting the resistive touch screen to be radiated by a UV lamp having a power of 40W to 60W until a hardened layer with bacteria inhibition capability having a thickness of about several micrometers is formed on the resistive touch screen (i.e., hardening treatment finished). In an experiment conducted by the present inventor for comparing the produced resistive touch screen of the invention with a prior resistive touch screen without being subjected to a bacteria inhibition process, E. coli having a density of 1 million per square centimeter is disseminated on the screen of each of the above touch screens. The number of E. coli living on the screen of each of the above touch screens is counted after 24 hours. A result shows that the number of E. coli still living on the resistive touch screen of the invention is about 99% less than that at beginning of the experiment (i.e., 99% reduction). As comparison, the number of E. coli still living on the prior resistive touch screen without being subjected to a bacteria inhibition process is about the same as that at beginning of the experiment. In conclusion, the touch screen of the invention can effectively inhibit the growth of bacteria thereon.

While the invention herein disclosed has been described by means of specific embodiments, numerous modifications and variations could be made thereto by those skilled in the art without departing from the scope of the invention set forth in the claims.

## Claims

1. A method for manufacturing a touch screen having a bacteria inhibition layer, whereby the substrate of the touch screen is a PET film, the method comprising:
uniformly dispersing particles of nano metal material in a solution to be applied to a surface treatment;
evenly spray coating the solution on a screen of the touch screen; and
subjecting the solution coated on the screen of the touch screen to a heat treatment until solvent in the solution is completely evaporated so that the particles of the nano metal material are densely adhered to the screen of the touch screen to form a bacteria inhibition layer thereon, wherein the solution has a concentration of nano metal material of 20ppm to 500ppm and that the nano metal material has particles having a diameter in the range of about 1 nm to 100nm,
**characterized in that**
a hardening treatment is performed by:
uniformly dispersing particles of nano metal material weighted 0.1g and having a diameter of about 10nm in methyl ethyl ketone weighted 100g for preparing a required dispersing solution;
adding an UV hardener mainly consisting of polybutene acrylate monomers weighted 900g in the dispersing solution;
uniformly mixing them; evenly spray coating the solution on screen of the resistive touch screen; subjecting the screen of the resistive touch screen to an IR heating for about 5 minutes until solvent in the solution is completely evaporated; and
subjecting the resistive touch screen to be radiated by a UV lamp having a power of 40W to 60W until a hardened layer with bacteria inhibition capability having a thickness of about several micrometers is formed on the resistive touch screen.

2. The method of claim 1, wherein the nano metal material is selected from gold (Au), silver (Ag), copper (Cu), zinc (Zn), or platinum (Pt).

3. The method of claim 1, wherein the nano metal material is selected from a combination or compound of gold (Au), silver (Ag), copper (Cu), zinc (Zn) or platinum (Pt).

4. The method of claim 1, 2 or 3, wherein the metal material has biochemical activation, is capable of penetrating cell membrane of a bacteria for damaging enzyme and killing the same naturally.

## Patentansprüche

1. Verfahren zur Herstellung eines Touchscreens (berührungsempfindlichen Bildschirms) mit antibakterieller Beschichtung, dessen Substrat eine PET-Folie ist, mit folgenden Schritten:
Homogenes Dispergieren von Nanoteilchen eines Metallwerkstoffs in einer mit einer Oberflächenbehandlung aufzubringenden Lösung;
gleichmäßiges Aufsprühen der Lösung auf den Touchscreen; und
Behandeln der auf den Touchscreen aufgetragenen Lösung mit Wärme, bis das Lösungsmittel der Lösung vollständig verdampft ist, so dass die Nanoteilchen des Metallwerkstoffs dicht am Touchscreen haften und auf ihm eine antibakterielle Schicht bilden, wobei die Konzentration der Nanoteilchen des Metallwerkstoffs in der Lösung 20 ppm bis 500 ppm beträgt und die Nanoteilchen des Metallwerkstoffs einen Durchmesser im Bereich von etwa 1 nm bis 100 nm haben,
**gekennzeichnet durch** eine Härtebehandlung derart, dass man
zur Zubereitung einer erforderlichen Dispersionslösung 0,1 g Nanoteilchen des Metallwerkstoffs mit einem Durchmesser von etwa 10 nm in 100 g Methylethylketon dispergiert;
der Dispersionslösung einen UV-Härter zugibt, der hauptsächlich aus 900 g Polybutenacrylatmonomeren besteht;
gleichförmig mischt und die Lösung gleichmäßig auf den Widerstands-Touchscreen sprüht; den Widerstands-Touchscreen etwa 5 min mit IR-Strahlung erwärmt, bis das Lösungsmittel in der Lösung vollständig verdampft ist; und den Widerstands-Touchscreen mit einer UV-Lampe mit 40 W bis 60 W Leistung bestrahlt, bis auf dem Widerstands-Touchscreen eine gehärtete, antibakteriell wirkende Schicht von etwa mehreren Mikrometern Dicke ausgebildet ist.

2. Verfahren nach Anspruch 1, bei dem der Metallwerkstoff der Nanoteilchen aus der Gruppe Gold (Au), Silver (Ag), Kupfer (Cu), Zink (Zn) oder Platin (Pt) gewählt ist.

3. Verfahren nach Anspruch 1, bei dem der Metallwerkstoff der Nanoteilchen aus Kombinationen oder Verbindungen von Gold (Au), Silber (Ag), Kupfer (Cu), Zink (Zn) oder Platin (Pt) gewählt ist.

4. Verfahren nach Anspruch 1, 2 oder 3, bei dem der Metallwerkstoff biochemisch aktiv wirkt und die Zellmembran eines Bakteriums durchdringen kann, um dort Enzyme zu schädigen und dieselbe auf natürlichem Weg abzutöten.

## Revendications

1. Procédé pour fabriquer un écran tactile comportant une couche d'inhibition bactérienne, selon lequel le substrat de l'écran tactile est un film en PET, lequel procédé comprend :
la dispersion uniforme de particules de nanomatériau métallique dans une solution à appliquer pour un traitement de surface ;
l'application uniforme par pulvérisation de la solution sur un écran de l'écran tactile ; et
l'exposition de la solution appliquée sur l'écran de l'écran tactile à un traitement par la chaleur jusqu'à ce que le solvant de la solution soit complètement évaporé, de telle sorte que les particules du nanomatériau métallique adhèrent densément à l'écran de l'écran tactile pour former sur celui-ci une couche d'inhibition bactérienne, dans lequel la solution a une concentration en nanomatériau métallique de 20 ppm à 500 ppm et le nanomatériau métallique comporte des particules ayant un diamètre compris entre environ 1 nm et 100 nm,
**caractérisé en ce qu'**un traitement de durcissement est exécuté par :
dispersion uniforme de particules de nanomatériau métallique pesées à 0,1 g et ayant un diamètre d'environ 10 nm dans de la méthyléthylcétone pesée à 100 g pour préparer une solution de dispersion requise ;
ajout d'un durcisseur sous UV composé principalement de monomères d'acrylate de polybutène pesé à 900 g dans la solution de dispersion ;
mélange uniforme de ceux-ci ; application uniforme par pulvérisation de la solution sur l'écran de l'écran tactile résistif ; exposition de l'écran de l'écran tactile résistif à un chauffage par infrarouges pendant environ 5 minutes jusqu'à ce que le solvant de la solution soit complètement évaporé ; et exposition de l'écran tactile résistif au rayonnement d'une lampe à ultraviolets ayant une puissance de 40 W à 60 W jusqu'à ce qu'une couche durcie ayant une capacité d'inhibition bactérienne d'une épaisseur de plusieurs microns environ se forme sur l'écran tactile résistif.

2. Procédé selon la revendication 1, dans lequel le nanomatériau métallique est choisi parmi l'or (Au), l'argent (Ag), le cuivre (Cu), le zinc (Zn) et le platine (Pt).

3. Procédé selon la revendication 1, dans lequel le nanomatériau métallique est choisi parmi une combinaison ou un composé d'or (Au), d'argent (Ag), de cuivre (Cu), de (Zn) ou de platine (Pt).

4. Procédé selon la revendication 1, 2 ou 3, dans lequel le matériau métallique a une activation biochimique et est capable de pénétrer à travers la membrane cellulaire d'une bactérie pour endommager ses enzymes et tuer celle-ci naturellement.
